# EUROPEAN PATENT SPECIFICATION

(11) **EP 0 371 437 B1**
(45) Date of publication and mention of the grant of the patent: **21.02.1996**
(21) Application number: 89121895.0
(22) Date of filing: 28.11.1989
(51) Int. Cl.: C12Q 1/68, G01N 33/53, C07H 21/04

(54) **Method and reagent combination for determining nucleotide sequences**
Verfahren und Reagenzkombination zur Bestimmung von Nukleotidsequenzen
Procédé et combinaison de réactif pour déterminer des séquences nucléotides

(30) Priority: 29.11.1988 US 277643
(43) Date of publication of application: 06.06.1990
(73) Proprietor: ORION-YHTYMÄ OY, SF-02101 Espoo (FI)
(72) Inventor: Söderland, Hans Erik, 02940 Espoo (FI); Syvänen, Ann-Christine Elizabeth, 00270 Helsinki (FI)
(74) Representative: VOSSIUS & PARTNER

(56) References cited:
- WO-A-86/05815
- WO-A-89/09282
- WO-A-89/12063
- GB-A- 2 202 328
- PROCEEDINGS OF THE NATIONAL ACADEMY OF SCIENCES USA, vol. 85, January 1988,
- pages 544-548; D.R. ENGELKE et al.: "Direct sequencing of enzymatically amplified human genomic DNA"
- NATURE, vol. 330, 26th November 1987, pages 384-386; C. WONG et al.: "Characterization of beta-thalassaemia mutations using direct genomic sequencing of amplified single copy DNA"
- MEETING ON THE POLYMERASE CHAIN REACTION: METHODOLOGY AND APPLICATIONS, 3rd - 7th April 1989; & J. CELL. BIOCHEM., suppl. 0, (13 part E), 1989, page 298, New York, US; L.G. MITCHELL et al.: "Affinity generation of single stranded DNA following the polymerase chain reaction: application to dideoxy sequencing"
- NUCLEIC ACIDS RESEARCH, vol. 16, no. 7, 1988, pages 3025-3028, IRL Press Ltd, Oxford, GB; S. STAHL et al.: "Solid phase DNA sequencing using the biotin- avidin system"

## Description

The present invention relates to a method and a reagent combination for determining the nucleotide sequence over a defined polynucleotide region, and to the use of this method in identifying genetic variations.

The genetic information of living organisms is carried in the nucleotide sequence of their genome. In the process of gene expression the nucleotide sequence is translated to amino acid sequences, i.e. proteins. Minor changes in the nucleotide sequence, even a single base substitution, may result in an altered protein product. The altered quality or quantity of given proteins changes the phenotype (i.e. the observable characteristics) of the organism or the cell, which for instance may be observed as a development of a disease. It would therefore be significant if changes in nucleotide sequences in the genome of living organisms could be determined accurately and with such efficiency that large numbers of samples could be screened. This would afford opportunities for diagnosis of hereditary predispositions or diseases, detection of somatic mutations in cancer, and selection of cells and strains for plant and animal breading.

The detection of changes in the nucleotide sequence can be done in different ways. A method yielding very convincing results is the actual determination of the nucleotide sequence over the region of the genome containing the gene of interest. Compared to nucleic acid hybridization methods, the method of determination of the nucleotide sequence makes it unnecessary to know the actual mutation. It is only necessary to know the location of the region of interest and to compare this region in a sample with the same region in a "normal" standard.

Two basic approaches are in use for nucleotide sequence determination. These are the chemical method of Maxam-Gilbert and the chain termination method of Sanger. Both methods are well described in contemporary biochemistry text books. Common to these two methods is the requirement for submicrogram amounts of relatively pure target polynucleotide to be sequenced. In most cases this target polynucleotide is cloned by genetic engineering methods into suitable vectors for biological amplification in order to obtain sufficient quantities of nucleic acid polymer for analysis. The repeated replication reaction (see Kleppe et al., J. Mol. Biol. 56 (1971), 341-361) has provided another way of amplifying given polynucleotide regions in vitro. The method has later been applied by Mullis et al. (see EP 200,362). While this method provides substantial advantages in speed and efficiency over the cloning method of producing adequate quantities of DNA, it does not provide a product which is directly suited for sequencing. Instead, the amplified region needs to be carefully purified e.g. by electrophoresis or ultrafiltration, from the amplification reaction mixture which if present during the sequencing process would be deleterious for the sequencing. These purification procedures tend to be time consuming arguing against their use in routine analysis.

Furthermore, GB-A-2 202 328 discloses a method for assaying nucleic acids by means of hybridization techniques after amplification of the target nucleic acid sequence. However, this method only allows to detect the presence or absence of a sequence within a sample. Engelke et al., Proc. Natl. Acad. Sci. USA 85 (1988), 544-548 reports on the sequencing of amplified human genomic DNA after carrying out a polymerase chain reaction wherein, however, all reactions are carried out in solution. Wong et al., Nature 330 (1987), 384-386 describes the characterisation of a β-thalassaemia mutation by direct genomic sequencing of a PCR-amplified DNA by the same method as described in Engelke et al., supra. Stahl et al., Nucleic Acids Res. 16 (1988), 3025-3028 discloses a method for solid-phase DNA sequencing using the biotin-avidin system. The method involves enzymatic incorporation of biotin-dUTP into a plasmid containing the target DNA, linearisation of the plasmid by a restriction enzyme, binding of the cleaved and biotin-labelled plasmid to avidin agarose, melting of the double strand and after primer annealing sequencing by the Sanger technique.

Thus, the technical problem underlying the present invention is to provide a method which permits a fast, reliable, simple and optionally automatic detection of changes in a nucleotide sequence which is well adapted for use as a routine diagnostic tool. This technical problem is solved by providing the method of the present invention and the kits to be used therein. These embodiments are characterized in the claims. When applying the method of the present invention, there is no necessity for cloning the target polynucleotide into specific vectors. Isolation of enzymatically amplified target polynucleotide is simple and there is no work consuming purification steps e.g. ultrafiltration or gel electrophoresis. Furthermore the sequencing reactions are performed on immobilized templates, which provides advantages in process control and possibilities for automation and simultaneous sequencing of both strands of target polynucleotide.

The method of the invention for detection of changes in a nucleotide sequence is based on the determination of the nucleotide sequence over the region of interest. In accordance with the invention, the first step of this process involves obtaining an amplified DNA sample in which at least one attachment moiety has been introduced into at least one strand of specific target polynucleotide by a modified polymerase chain reaction. This can be accomplished by combining a sample containing the target nucleic acid polymer with a mixture of nucleotide monomers (dATP, dCTP, dGTP, and dTTP), a DNA polymerase, the first and second amplification primers. Each of the amplification primers comprises an oligonucleotide having a region that is complementary to the hybridizes with a different strand (i.e., one or the other of the two complementary strands) of the target nucleic acid polymer and that is effective as a primer for DNA polymerization by the DNA polymerase. At least one of the amplification primers further comprises a first attachment moiety bonded to the oligonucleotide. The resulting amplification mixture is repeatedly cycled between a first condition under which DNA polymerization occurs and a second condition under which denaturation of double stranded DNA to single stranded DNA occurs for a number of cycles sufficient to produce copies of the target nucleic acid polymer in a quantity suitable for use in a DNA sequencing procedure to form an amplified sample comprising copies of the target nucleic acid sequence bonded to the attachment moiety. A third incubation condition under which annealing of single to double stranded DNA occurs, particularly annealing of the single stranded amplification primers to the single stranded template DNA, may advantageously be incorporated before each polymerization step.

After amplification, the strands of target nucleic acid polymer carrying an attachment moiety are captured using a solid matrix coated with an attachment site to which the attachment moiety or a modification thereof can bind. In this way, the strands of target nucleic acid become bound to the solid matrix and can be readily separated with the solid matrix from unbound reactants.

The purified copies of the target polynucleotide are denatured either before immobilization or following separation of the solid matrix from the unbound reactants, and the sequence of the amplified target polymer is determined. For example, chain termination sequencing reactions can be initiated on the denatured copies of the target polynucleotide, which are now acting as templates. The newly synthesized sequencing reaction products are then released from the template and separated and detected by size.

The present invention also encompasses a reagent combination or kit for practising the method of the invention. While the precise packaging will depend on the combinations of attachment moieties and attachment sites selected, such a kit will in general include an amplification primer having an attachment moiety included, a support adapted to immobilize the amplification primer and a sequencing primer.

The method of the present invention is useful for determining in a target nucleic acid at least one potential nucleotide variation, said variation being a nucleotide replacement, deletion or insertion.

Thus, the method of the present invention is applicable in forensic analysis or the analysis of hereditary predispositions or diseases. In some of these analyses the nucleotide variation is a somatic mutation.
From the above it is evident that the method of the present invention can also be applied in selecting cells, strains or species in plant or animal breeding or for selecting microorganisms displaying a desired genotype.
The kits of the present invention are suitable for performing the above mentioned methods.

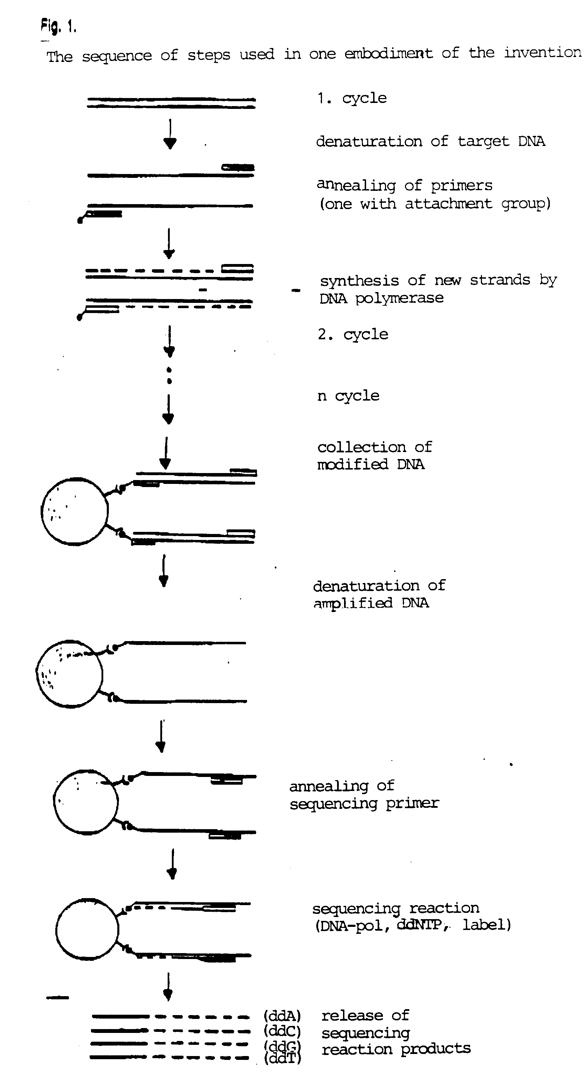

Fig. 1 is a schematic drawing of the sequence of steps used in one embodiment of the invention; and
Fig. 2 is a schematic drawing of possible regions of complementarity between a target nucleic acid and a sequencing primer.

The present invention discloses a method to determine the nucleotide sequence of previously defined regions in complex nucleic acid mixtures. In a preferred embodiment, two known basic methods are utilized: the chain-termination sequencing procedure of Sanger and the repeated replication reaction (RRR). The invention also utilizes the affinity based hybrid collection procedure described in GB-A-2202328. The novel combination of these methods in the present invention yields a facile and efficient process for sequencing nucleic acids which is well suited to the detection of genetic defects.

In accordance with the invention, a small sample of target nucleic acid is first amplified using a repeated replication reaction (RRR) in which one or both of the amplification primers are modified to include an attachment moiety, for example, one member of an affinity pair. The target sequence of interest is amplified with a suitable number of cycles of this modified RRR-reaction. Then the sample may be treated to render double-stranded nucleic acids single stranded, whereafter all molecules modified with a given attachment moiety are bound to a solid matrix coated with a complementary attachment site, e.g. the other component of an affinity pair. The matrix is then washed to remove all unbound material. It is also possible to attach amplified DNA to the matrix before denaturation. In that case, the denaturation of double-stranded adsorbed nucleic acid molecules to single stranded form would be done after immobilization (attachment). This approach also makes it possible to elute the essentially pure complementary strand even though it may not have been synthesized with an attachment moiety.

Independent of the stage at which the denaturation step is done, the result is a solid support onto which single-stranded nucleic acid is bound from one end. This solid support, preferably microbeads, is then introduced into the reaction mixtures of the chain termination sequencing process, one reaction mixture for each nucleotide, A,C,G, or T. The polymerization step is allowed to proceed on the beads. Then the beads may be washed if desired, whereafter the polymerization product is eluted from the beads and analyzed by gel electrophoresis as described for a number of manual, semimanual or automated sequencing protocols. In a modification of this process the essentially pure complementary strand eluted from matrix bound modified strand is used as the sequencing template. If both amplification primers are modified with a different attachment moiety and the resulting amplified DNA is "attached" to different physically separable matrices, both strands can be introduced into the same sequencing reaction mixture and the matrices to which they are attached can be separated from each other as late as just prior to the final denaturation and gel electrophoresis. This possibility of sequencing both strands simultaneously with separation of the two not occurring unit just prior to electrophoresis decreases the amount of sequencing work by almost a factor of two. The different modes of practising the present invention are now detailed.

### (a) Modification of target nucleic acid fragments with attachment moieties.

The source of the target nucleic acid (DNA or RNA) to be analyzed by the method of the invention can be any human cell, animal cell, plant cell or microbe. The target nucleic acid can be isolated from biological samples by conventional nucleic acid purification methods but according to the present invention it is also possible to use unpurified biological samples directly.

In the method of the invention attachment moieties are introduced into the target polynucleotide by the modified repeated replication reaction with the aid of two oligonucleotide primers per region to be amplified. At least one of these "amplification" primers has been modified with an attachment moiety. For purposes of this application and the claims hereof, an affinity moiety is a component having affinity for another component which forms an affinity pair by preferentially and selectively binding with that other component. For example, biotin/avidin or streptavidin, complementary polynucleotides, including homopolynucleotides such as poly dA/poly dT and poly dG/poly dC, hapten/antibody, and metal/chelate, are such affinity pairs. Any component pairs with strong affinity for each other can function as an affinity pair. An attachment moiety is an affinity moiety or a moiety providing a site for the attachment of an affinity moiety.

The oligonucleotide primers may be synthesized by standard chemical methods. Also recombinant DNA techniques may be used for the preparation of primers. The size of the primers is preferably between 14 and 40 bases, but primers of at least 8 bases or considerably longer than 40 bases may also be used. The primers are modified with the attachment moieties using chemical or enzymatic or any other methods. Preferably the attachment moiety is attached to the 5' end of the primer. Other sites of attachment are also possible, provided that the base pairing property of the primer and its primer function are retained.

In one embodiment of the method of the invention two modified primers are used. The primers must in this case be modified with different attachment moieties.

In the method of the invention the amplification primers, one or both modified, the target nucleic acid, mononucleoside triphosphates and a nucleic acid synthesizing enzyme, such as DNA polymerase, are combined. Repeated cycles of denaturation of the target, annealing of the primers to the target strands and enzymatic elongation of the primers are carried out according to any repeated replication reaction protocol for as many cycles as necessary to obtain a quantity of nucleic acid sufficient for use in the DNA sequencing reaction to be used. For use in chain termination sequencing reactions, at least 10¹⁰ molecules of DNA are preferably obtained.

As a result of the process, copies of the original target polynucleotide, now modified with attachment moieties, are produced by incorporation of the modified primers into the newly synthesized polynucleotide molecules. When only one of the primers is modified, polynucleotide molecules with one strand modified with an attachment moiety are synthesized. The use of two differently modified primers, each adapted to hybridize with a different strand of the target nucleic acid, produces polynucleotide molecules with one strand modified with one attachment moiety and the other strand modified with a different attachment moiety. A special case is obtained when one of the primers is modified with a polynucleotide attachment moiety. In this case the other strand becomes modified with the complementary sequence during the RRR-process which can then serve as an attachment moiety for the second strand. The size and amount of the modified target DNA produced is limited only by the capacity of the repeated replication reaction.

### (b) Isolation of single stranded target polynucleotide

After synthesis the modified target polynucleotide is captured on a solid matrix, to which an attachment site has been attached. For purposes of this application and the claims hereof, an attachment site is a component of an affinity pair which is selected so as to preferentially and selectively bond with the affinity moiety used as or subsequently bonded to the attachment moiety incorporated in the amplification primer. When one strand of the target polynucleotide is modified with an affinity moiety, one solid matrix is used, and when both strands are modified, two different solid matrices are used. Preferably the two matrices are selected such that they can be physically separated from each other. Alternatively, the capturing of the two differently modified strands can be done sequentially on two different solid matrices.

The solid matrix can be of any format, such as beads, microparticles, the surface of a microtitration well or a test tube, a dipstick or a filter. The material of the matrix may for instance be polystyrene, cellulose, latex, nitrocellulose, nylon, polyacrylamide, dextran or agarose. The only prerequisite for the material of the matrix is that the attachment site can be attached to it. The binding capacity of the matrix should enable capture of a sufficient amount of the modified target polynucleotide as well as the excess of the modified primer present, which will also be captured. However,the use of a matrix which easily can be divided into portions is preferred, e.g. a large number of matrix particles of lesser binding capacity as opposed to a few matrix particles of very high capacity.

The modified target nucleic acid can be brought into contact with the affinity matrix directly in the reaction mixture in which the amplification was carried out. The mixture may also be diluted, or its composition may be changed to obtain conditions favorable for the reaction between the components of the affinity pair.

When two affinity pairs are used the target polynucleotide is preferably denatured before the affinity capture reaction to enable separate collection of the two strands on different affinity matrices. If these two different affinity matrices can be physically separated from each other e.g. after sequencing reaction stage, the two matrices can be handled as one in the capturing stage and in the sequencing reaction stage. When the target is modified in one strand only, it is optional to denature the target before the capturing reaction.

The attachment moieties of the target polynucleotide are allowed to react with the immobilized complementary attachment site for a sufficient time period to recover a substantial portion of the amplified target nucleic acid, which may vary depending on the kinetics of the binding reaction. In the case that the attachment moiety is not directly bondable to the attachment site this will further involve the introduction of a linking moiety, a molecule with separate binding sites for the attachment moiety and the attachment site which can form a link between the two. After the capturing reaction the bound target polynucleotide is separated from unbound material, including excess mononucleoside triphosphates, unmodified primer, enzyme, salts etc. by washing the matrix under suitable conditions. The separation procedure depends on the physical form of the matrix, and may for instance be centrifugation, magnetic separation, a column procedure or rinsing of the surface of the matrix or combinations thereof.

After the washing procedure, the matrix is treated under conditions that render the bound target polynucleotide single stranded. Any conditions which denature double stranded polynucleotide, such as heat, alkali or enzymes, but do not affect the bonds between the components of the affinity pair, may be used. This step is compulsory if the target polynucleotide was not denatured before the capturing reaction. When denaturation was done before the capturing reaction this step is advantageous, but optional, to ensure that target molecules that may have reannealed during the capturing step are denatured. The exception, of course, is the case in which the attachment moiety and attachment site are complementary nucleic acid strands. In this case, denaturation must precede attachment since the link to the support would otherwise be lost.

### (c) The sequencing reaction

According to the present invention the sequence determination of the isolated amplified polynucleotide fragments may be performed using the chain termination method. The method is based on the use of deoxynucleotide analogues which are randomly incorporated by a polynucleotide synthesizing enzyme into a growing polynucleotide strand to give specific chain termination. Chain termination can also be achieved using very low concentrations of one of the deoxynucleoside triphosphates.

The necessary components of the chain termination sequencing reaction are a sequencing primer, a polynucleotide synthesizing enzyme, the chain terminating dideoxynucleotides, deoxynucleotides and a detectable label. According to the present invention the different standard laboratory protocols for sequencing are applied to sequencing the immobilized target polynucleotide. The order of addition of the components of the sequencing reaction may vary, depending on the affinity matrix used and on the chosen protocol.

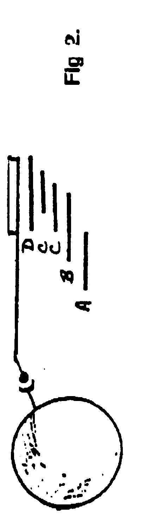

As shown in Fig. 2, the sequencing primer may be distinct from or equal to the primer used in the RRR amplification reaction. If it is distinct from the amplification primer it may be completely located between the region of interest in the target nucleic acid polymer and the 3' end of the amplification primer (A), or it may be partially overlapping the amplification primer in such a way that its 3' end anneals at least one nucleotide, but preferably more than 3 nucleotides of the 5' end of the target nucleic acid polymer (B). The sequencing primer may also fall entirely within the amplification primer (C) or be the same as the amplification primer (D).

The choice of sequencing primer depends on the purpose of the analysis. A primer distinct from the RRR primer will increase the specificity of the sequencing reaction, because sequencing of wrong fragments possibly produced in the RRR is avoided. Thus, a sequencing primer distinct from the amplification primer is preferred. On the other hand, more prior information of the sequence of the amplified fragment is needed to make such a primer.

The label for detection of the fragments produced in the sequencing reaction can be introduced by using a labelled sequencing primer. The label may be situated at the 5'-end or internally. It may be an isotope, such as ³P, or a fluorescent group, or any other detectable label, or a group providing a site for later attachment of a detectable label. The label can also be introduced using deoxynucleotide derivatives labelled with an isotope, such as ³⁵S or ³P, or with fluorescent or other detectable labels. The labeling moiety is incorporated in an amount such that it is incorporated in substantially all of the nucleic acid synthesized, even very short fragments.

The matrix carrying the polynucleotide fragment is suspended into a suitable solution containing the sequencing primer. The primer is preferably added in equal or excess molar concentration to the target polynucleotide. The primer is allowed to anneal to the target polynucleotide at suitable temperature and for a sufficient time depending on the size and amount of the primer used. It should be noticed that when both strands of target polynucleotide are modified and bound to different affinity matrices, which can be physically separated from each other and handled now as one, two different sequencing primers are used.

If a dividable affinity matrix, such as beads, is used it is convenient to divide the sample after annealing of the primer into four parts of the individual sequencing reactions. If an undividable affinity matrix, such as a microtitration well is used, four parallel reactions are prepared at the affinity-capturing step. It is also possible to carry out the four reactions sequentially on the same carrier by eluting the product of each reaction before carrying out the next one. In certain cases enough information is obtained using only one, two, or three reactions. For example, if a genetic defect is known to arise from a point mutation at a single specific site which is normally C, it may be sufficient to run only the C reactions since the actual identity of a non-C nucleic acid residue at this point is not needed. The relevant fact is that the residue is non-C.

After annealing, a polynucleotide synthesizing enzyme, such as E. coli DNA polymerase I or T7 DNA polymerase, and mixtures containing the chain terminating dideoxynucleotides, deoxynucleotides, and if unlabelled primers were used, labelled deoxynucleotides at suitable ratios which depend on the enzyme used and on the size of the target, are added. The polymerization reaction is allowed to proceed for a sufficient time period to achieve a substantially complete distribution of fragment sizes at a temperature that depends on the enzyme and conditions used. Finally, a chase reaction with deoxynucleotides is carried out to complete the synthesis of the polynucleotide strands which have not been terminated. The reaction is then stopped.

In the method of the invention the affinity matrix may be washed at this stage to remove the components of the sequencing reaction. The composition of the mixture may also thus be changed to obtain the most favorable conditions for the analysis of the synthesized fragments. If both strands were modified and bound to two different affinity matrices, the matrices are physically separated at this stage, e.g., by a magnetic field. The target DNA is rendered single stranded and the synthesized fragments from the four sequencing reactions are separated by electrophoresis or another suitable method. In the method of the invention the affinity matrix can be removed before electrophoresis, or when the affinity matrix is a microparticle it can be loaded together with the sample into the slots of the gel for the electrophoresis step. This gives an advantage in sample handling. In the electrophoresis step the size distribution of the fragments is analyzed. The detection procedure may vary depending on the label used. With isotopes the gel is prepared by fixing and drying and exposed to an x-ray film according to standard procedures. With fluorescent labels the fragments are detected using automatic equipment developed for this purpose. The size distribution of the polynucleotide fragments produced by the sequencing reaction produces a pattern called a "sequencing ladder" which gives the nucleotide sequence of the target polynucleotide.

Reagents for use in practising the method of the invention may be packaged in kit form. For example, kits might be prepared comprising packaged combinations of one or two amplification primers which include attachment moieties and the corresponding solid supports and a sequencing primer. Kits might also be prepared which further include a reference means for comparison of the determined sequence of the nucleic acid polymer with the sequence of a standard. Examples of reference means include package inserts showing the sequencing ladder obtained from sequencing of the standard, or a sample of the standard itself. If the standard is supplied, it advantageously has an attachment moiety attached to it so that it can be handled in a similar manner to the target for sequencing.

The arrangement of the reagents within containers of the kit will depend on the specific reagents involved. Of course, each reagent can be packaged in an individual container, but various combinations may also be possible. For example, if the attachment moiety and the attachment site used will not bind to each other under the conditions of the RRR-reaction, both can be provided in a single container. This might be the case, for example, if a linking moiety was used.

The present invention is illustrated more specifically with the following examples, which are not intended to limit the scope of the invention.

### Example 1

Determination of the nucleotide sequence of a biotinylated cytomegalovirus DNA fragment immobilized on avidin-microparticles

### Synthesis of the Primers

The primers were synthesized on an Applied Biosystems 381A DNA synthesizer by the methoxy phosphoramidite method. The primers (denoted 25A and 25B) were 25-mers with the following nucleotide sequence:

25A: 5'-TCG CAA GCT CTT TCC CGG CCT GGC T
25B: 5'-GCT CTG CGC GAA CAT GTA GTC GGC C

A 5'-terminal aminogroup was added to the 25A-primer as the last step in the synthesis using the aminolink II reagent (Applied Biosystems, P/N 400808). The aminogroup was biotinylated with sulfo-NHS-biotin (Pierce Chemical Co.). The biotinylated oligonucleotide was purified by HPLC on a reversed phase C-18 column.

### The Target DNA

The target DNA was a fragment of the long unique region of the cytomegalovirus (CMV) genome at map position 0.30-0.35, i.e. the 12.2 kb Hind IIIL-fragment of AD 169 CMV DNA(ATC VR-538) cloned in the pAT153 vector. The target plasmid was linearized with the restriction enzyme Eco RI. The primers flank a 115 bp region in the CMV Hind IIIL fragment.

### Primer Extension Reaction-Amplification

Biotin was introduced into a 165 base pair fragment of the CMV DNA by 25 cycles of amplification by the repeated replication reaction (RRR) using the 5'-biotinylated primer 25A and the unmodified primer 25B. The amplification was carried out with 3 x 10⁵ molecules of the linearized CMV Hind IIIL fragment, 100 pmol each of the primers, 200 µM each of the four mononucleoside triphosphates (dATP, dCTP, dGTP, dTTP) in 100 µl of a solution of 10 mM Tris-HCL, pH 8.35, 50 mM KCl, 1.5 mM MgCl₂ and 0.1 mg/ml gelatin in 0.5 ml Eppendorf tubes under a layer of viscous paraffin. The target DNA was boiled for 5 min. in the reaction mixture to render it single stranded, after which 2U of Thermus aquaticus DNA-polymerase (New England Biolabs) was added. The tubes were then processed for 25 cycles as follows: Primer annealing at 55°C for 1 min., extension of the primers at 72°C for 3 min., denaturation of the template at 95°C for 1 min 15 sec.

### Capture of the Biotinylated Amplified DNA on Avidin-coated Polystyrene Particles.

A 25 µl sample, which contained about 10¹⁰ molecules, i.e. 0.02 pmoles, of the amplified DNA-fragment, which was dcuble stranded and contained a 5'-biotin in one strand, was collected from the reaction mixture by incubation with 5 µl of a 5% solution of avidin-coated polystyrene particles (0.8 µm, Pandex Laboratories) for 1 hour at room temperature. The beads were collected by centrifugation in an Eppendorf centrifuge for 3 min. and washed three times by vortexing with 1 ml of phosphate buffered saline (PBS), pH 7.5, containing 0.1% Triton®-X-100, followed by centrifugation as above. The bound DNA was denatured by treatment with 200 µl of 0.15 M NaOH for 15 min. at 37°C, the particles were collected and washed twice with 1 ml of 0.1% Triton®-X-100, and finally once with 1 ml of 0.01% Triton®-X-100.

### Sequencing of the Immobilized DNA Fragment

The microparticles carrying the DNA fragment were suspended into 10 µl of 40 mM Tris-HCl, pH 7.5, 20 mM MgCl₂, 50 mM NaCl, 100 µg/ml bovine serum albumin, containing 0.06 - 0.08 pmoles of the 25B primer. The primer was allowed to anneal for 10 min. at 55°C followed by 30 min. at 37°C. Then 2 µl of ³⁵S-labelled dCTP (600 Ci/mmol; Amersham) and 1 µl (5 units) of E. coli DNA polymerase (the Klenow fragment; Boehringer Mannheim) were added. The suspension was divided into four test tubes, 2.5 µl in each, for the individual sequencing reactions. 2 µl of deoxy- and dideoxynucleotide mixtures were added to yield the following final concentrations:
- A-reaction:: 125 µM dGTP, dTTP, 16 µM dATP, 250 µM ddATP
- C-reaction:: 125 µM dGTP, dTTP, dATP, 20 µM ddCTP
- G-reaction:: 125 µM dTTP, dATP, 16 µM dGTP, 200 µM ddGTP
- T-reaction:: 125 µM dGTP, dATP, 16 µM dTTP, 500 µM ddTTP.
The reaction was allowed to proceed for 20 min. at room temperature. Then 2 µl of a 2 mM solution of the four dNTPs were added, and the chase reaction was allowed to proceed for 20 min. at room temperature. The reaction was stopped by adding 4 µl of 95% formamide, 20 mM EDTA, 0.05% bromophenol blue, 0.05% xylene cyanol. The samples were heated for 3 min. at 98°C. The tubes were centrifuged and 3 µl of the supernatant was loaded on an 8% polyacrylamide sequencing gel. The samples were electrophoresed, the gel removed, fixed and exposed to an x-ray film according to standard procedures. After development clear sequencing ladders were observed.

### Example 2

The whole procedure was carried out identically as in Example 1, with the exception that the samples from the sequencing reaction were loaded directly onto the gel without prior removal of the microparticles. Sequencing ladders identical to those in Example 1 were obtained.

### Example 3

In this example ³P-labelled primers were used in the sequencing reactions. The procedure was carried out as in Example 1, with the following exceptions:

The sequencing primer (25B) was labelled using gamma-³P-ATP and polynucleotide kinase according to a standard procedure. 2 pmoles of oligonucleotide primer and 14 pmoles of gamma-³P-ATP (> 5000 Ci/mmol; Amersham) were used per labelling reaction. Primers with specific activities of 10⁷ cpm/pmol were obtained.

For the sequencing reaction the microparticles carrying about 0.02 pmoles of the amplified DNA fragment were suspended in the sequencing buffer (see Example 1) containing 0.1 pmole of the labelled 25B-primer. The primer annealing reaction was as in Example 1. Then 1 µl (5 units) of DNA polymerase was added and the suspension was divided into four parts, 2.5 µl each. 2 µl of deoxy- and dideoxy-nucleotide mixtures were added to yield the following final concentrations:
- A-reaction:: 125 µM dCTP, dGTP, dTTP, 16 µM dATP and 250 µM ddATP
- C-reaction:: 125 µM dATP, dGTP, dTTP, 16 µM dCTP and 80 µM ddCTP
- G-reaction:: 125 µM dATP, dCTP, dTTP, 16 µM dGTP and 200 µM ddGTP
- T-reaction:: 125 µM dATP, dCTP, dGTP, 16 µM dTTP, 500 µM ddTTP.

The chain termination and chase reactions, as well as analysis of the produced fragments were carried out as in Example 1. Clear sequencing ladders were obtained also in this example.

### Example 4

In this example the RRR-primer (amplification primer) and sequencing pimer used were non-identical. The sequencing primer was located at a 78 base distance from the amplification primer toward the biotinylated 5'-end of the amplified fragment.

The PCR-amplification was carried out with the biotinylated primer 25A and a 25-mer primer, denoted 25C, with the following sequence:
5'-CAG GGT CAC TGA CAA CAG CCG CCC T. The primers 25A and 25C flank a 209 bp sequence in the CMV-genome.

The amplification and capturing procedures were as described in Example 1. The primer 25B was labelled with ³P and used as sequencing primer at the conditions described in Example 3. The obtained sequence was identical to that one obtained in Example 3.

### Example 5

All processes were carried out as in Example 1 except for the affinity-capturing step. In this example magnetic beads were used as the affinity matrix. The beads were 2 µm in diameter and had a paramagnetic nucleus with a spherical polystyrene shell coated with streptavidin. The beads (Dynabead 280-Streptavidin) were obtained from Dynal, Norway. Before use the beads were washed once with phosphate buffered saline (PBS).

As in Example 1, 25 µl of the sample containing the amplified DNA fragment was analyzed. 5 µl of a 4% suspension of the magnetic beads in PBS were added and the capturing reaction was allowed to proceed for 30 min. at 37°C in Eppendorf tubes in a rotating mixer. The beads were separated from the solution for 1 min. using a standard laboratory magnet and the solution was discarded. The beads were washed three times with 1 ml of PBS by vortexing followed by magnetic separation as above. The bound DNA was denatured by treatment with 200 µl of 0.15 M NaOH for 15 min. at 37°C. The beads were collected and washed twice with PBS and once with 40 mM Tris-HCl, pH 7.5, 20 mM MgCl₂, 50 mM NaCl. The sequencing of the immobilized DNA fragment was as in Example 1.

### Example 6

The RRR-amplification and capturing procedures were carried out as in Example 1. In this example the second strand of the amplified DNA fragment obtained at the denaturation step was sequenced.

The 200 µl 0.15 M NaOH supernatant containing the DNA-strand eluted from the affinity matrix was neutralized by the addition of 20 µl of 1.5 M acetic acid and 12 µl of 3 M sodium acetate (pH 7). The DNA was precipitated with ethanol and dissolved in 10 µl of the sequencing buffer (see Example 1) containing the sequencing primer. The sequencing procedure was carried out at the conditions specified in Example 1. Clear sequencing ladders were again obtained.

### Example 7

Identification of genetic polymorphism of the apolipoprotein E by sequencing immobilized biotinylated apolipoprotein E DNA fragments

This example is given to illustrate the conveniency of the method of the claimed invention for the diagnosis of genetic diseases. The method was applied to the analysis of the genetic variation of the apolipoprotein E (apo E). This protein plays an important role in the lipoprotein metabolism. Three common isoforms of apo E (E2, E3 and E4), encoded by three different alleles (ε2, ε3 and ε4), exist in the population. The genetic variation of these alleles is due to single base substitutions at amino acid positions 112 and 158. This genetic polymorphism of apo E may explain as much as 10 **%** of the individual variation of serum cholesterol levels and is thus of major clinical significance.

### Synthesis of the primers

Four primers (P1 -P4) were synthesized on an Applied Biosystems 381A DNA synthesizer. The nucleotide sequence of the primers and their location (given as nucleotide numbers) on the apolipoprotein E gene (Apo E) were:

P1: 5′-AAG GAG TTG AAG GCC TAC AAA T (3615 - 3637)
P2: 5′-TCG CGG GCC CCG GCC TGG TAC A (3893 - 3914)
P3: 5′-GAA CAA CTG AGC CCG GTG GCG G (3649 - 3671)
P4: 5′-GGA TGG CGC TGA GGC CGC GCT C (4012 - 4034)

A 5'-aminogroup was added to the primer P2 with the aminolink II reagent (Applied Biosystems). The amino group was biotinylated using sulfo-NHS-biotin (Pierce Chemical Co.) and purified by reversed phase HPLC. The sequencing primer (P3) was labelled with [gamma-³P]dATP and T4 polynucleotide kinase to a specific activity of 4 x 10⁶ cpm/pm.

### The DNA Samples

Venous blood samples were obtained from patients of known Apo E phenotype attending the Lipid Outpatient Clinic of the University Central Hospital of Helsinki. Leukocytic DNA was extracted according to standard procedures.

### Amplification

The DNA (100 ng per sample) was amplified with the P1 and P4 primers (final concentration 1 µM) in 100 µl of a solution of 0.2 mM each of dATP, dCTP, dGTP, dTTP, 20 mM Tris-HCl, pH 8.8, 15 mM (NH)₄SO₄, 1.5 mM MgCl₂, 0.1 % tween 20, 0.1 mg/ml gelatin and 2.5 units of Thermus aquaticus DNA-polymerase (Promega) in a DNA thermal cycler (Perkin-Elmer /Cetus) for 25 cycles of 1 min. at 96°C and 2 min. at 65°C. A small aliquot (3 µl of a 1:100 dilution) of this first amplification mixture was transferred to a second amplification. This second amplification was carried out at the conditions described above and directed by a pair of nested primers one of which was biotinylated (biotinylated P2 and P3).

### Affinity-capture of the Biotinylated Amplified Apo E DNA on Avidin-coated Polystyrene Particles

A 25 µl aliquot of the second amplification mixture was diluted to 50 µl with 0.15 M NaCl, 20 mM Na-phosphate buffer, pH 7.5 (PBS), after which 5 µl of a 5 % (w/v) suspension of avidin-coated polystyrene particles (0.8 µm, Baxter Healthcare Corp.) were added. The samples were kept at 20°C for 1 hour. The particles were collected by centrifugation for 2 min. in an Eppendorf centrifuge and were washed twice by vortexing with 1 ml of 15 mM NaCl, 1.5 mM Na-citrate, and twice with 1 ml of 0.1 % Tween^{®}20 in PBS. The particles were treated twice with 0.15 M NaOH for 15 min. at 37°C, followed by two washes with 0.1 % Tween®20 in 50 mM NaCl, 40 mM Tris-HCl, pH 7.5 and a final wash with 0.01 % Tween®20 in 50 mM NaCl, 40 mM Tris-HCl, pH 7.5.

### Nucleotide Sequencing of the Immobilized DNA Fragment

The particles carrying the DNA template were suspended in 10 µl of 50 mM NaCl, 20 mM MgCl₂, 40 mM Tris-HCl, pH 7.5, containing 0.5 - 1 pmol of the sequencing primer P2 or P3. The tubes were heated at 65°C for 2 min. and allowed to cool slowly to room temperature. One µl of 0.1 M dithiothreitol, 2.5 µl of H₂O and 2 µl (3.25 units) of T7 DNA-polymerase (Sequenase™, United States Biochemical Corp.) were added and the tubes were kept at 22°C for 3 min. An aliquot (3.5 µl) of the reaction mixture was transferred to four tubes containing 2.5 µl of chain termination mixture (80 µM dNTP, 8 µM ddNTP for the A and T reactions; 320 µM dNTP, 8 µM ddNTP for the C and G reactions). The tubes were incubated at 420C for 6 min. and the reaction was stopped by adding 4 µl of a solution containing 95 % formamide, 20 mM EDTA, 0.05 % bromophenol blue and 0.05 % xylene cyanol. The samples were heated at 80°C for 2 min. and the particles were spun down for 2 min. The products of the chain termination reactions were run on 6 % sequencing gels. The electrophoresis was carried out and the gel was processed according to standard procedures. The correct allelles were identified from the sequencing ladders obtained. In heterozygous samples both alleles were identified as superimposed sequencing ladders at the position of the nucleotide variation.

## Claims

1. A method for determining the sequence of a target nucleic acid polymer comprising:
(a) combining a sample containing the target nucleic acid polymer with a mixture of nucleoside triphosphates, a nucleic acid polymerase, and first and second amplification primers, each amplification primer comprising an oligonucleotide having a region that is complementary to and hybridizes with a different strand of the target nucleic acid polymer and being effective as a primer for nucleic acid polymerization by the nucleic acid polymerase to form an amplification mixture, wherein said first amplification primer further comprises a first attachment moiety bonded to the oligonucleotide;
(b) repeatedly cycling the amplification mixture between a first condition under which DNA polymerization occurs and a second condition under which denaturation of double stranded DNA to single stranded DNA occurs for a number of cycles sufficient to produce copies of the target nucleic acid polymer in a quantity suitable for use in a DNA sequencing procedure to form an amplified sample comprising copies of the target nucleic acid sequence bonded to the attachment moiety;
(c) adding to the amplified sample a plurality of first supports, each comprising a solid matrix and a first attachment site which is capable of immobilizing one strand of the target nucleic acid polymer through the first attachment moiety;
(d) allowing the first attachment sites to interact with the first attachment moieties such that the copies of the target nucleic acid in the amplified sample are immobilized on the first supports;
(e) separating the immobilized nucleic acids from the amplification mixture;
(f) denaturing the amplified nucleic acid sample to form single stranded nucleic acids; and
(g) determining the sequence of the immobilized single stranded nucleic acids.

2. A method according to claim 1, wherein the step of denaturing the amplified sample is performed prior to immobilizing the copies of the target nucleic acid on the support.

3. A method according to claim 1 or 2, wherein the sequence of the single stranded immobilized nucleic acids is determined using a chain termination method.

4. A method according to any one of claims 1 to 3, wherein said first attachment moiety and said first attachment site are components of an affinity pair.

5. A method according to claim 4, wherein the affinity pair is selected from the group consisting of biotin/avidin, biotin/streptavidin, poly-dA/poly-dT, poly-dG/poly-dC, hapten/antibody and metal/chelate.

6. A method according to any one of claims 1 to 5, wherein said second amplification primer comprises a second attachment moiety different from said first attachment moiety.

7. A method according to claim 6, further comprising the step of adding to the amplified sample a plurality of second supports comprising a support matrix and a second attachment site capable of immobilizing one strand of the target nucleic acid polymer through the second attachment moiety.

8. A method according to claim 6 or 7, wherein said second attachment moiety and said second attachment site are components of an affinity pair.

9. A method according to claim 8, wherein the affinity pair is selected from the group consisting of biotin/avidin, biotin/streptavidin, poly-dA/poly-dT, poly-dG/poly-dC, hapten/antibody and metal/chelate.

10. A method according to any one of claims 6 to 9, wherein said first and second supports are concurrently present in the amplified sample and are selected so as to be physically separable from each other.

11. A method according to claim 10, wherein one of said first and second supports are magnetic.

12. A method according to claim 10, wherein one or both of said first and second supports is a dipstick.

13. A method according to any one of claims 1 to 12, wherein the sequence-determination process includes the steps of
combining the single-stranded immobilized nucleic acids with a sequencing primer and a sequencing mixture comprising nucleoside triphosphates, one chain terminating nucleotide, a polynucleotide synthesizing enzyme and a labeling moiety which is incorporated during nucleic acid synthesis;
allowing polymerization to occur for a period of time sufficient for enough chain termination events to occur to permit determination of the sequence to form a partially polymerized sample;
adding a completion reagent comprising a mixture of nucleoside triphosphates to the partially polymerized sample;
allowing polymerization to occur in the completion reagent for a period of time sufficient to substantially complete DNA synthesis;
separating the completely polymerized sample from the completion reagent:
releasing the polymerized nucleic acids from the support;
denaturing the polymerized nucleic acids to render them single stranded;
analyzing the size of the single stranded nucleic acid polymers to determine the sequence of the target nucleic acid polymer.

14. A method according to claim 13, wherein the sequencing primer is an oligonucleotide selected from among oligonucleotides complementary with at least a portion of the amplification primer and oligonucleotides complementary with a portion of the target nucleic acid polymer which is located between the 3' end of the amplification primer and a region of interest in the target nucleic acid polymer.

15. A method according to any one of claims 1 to 14, further comprising the step of incubating the amplification mixture at a temperature selected to promote annealing of nucleic acids before each polymerization step.

16. A kit for use in determining the sequence of a target nucleic acid polymer comprising in packaged combination
(a) a first amplification primer comprising an oligonucleotide which is complementary to and hybridizes with a portion of the target nucleic acid polymer and which is effective as a primer for enzymatic nucleic acid polymerization and a first attachment moiety;
(b) a plurality of first supports, each comprising a solid matrix and at least one attachment site which is capable of immobilizing the oligonucleotide of the amplification probe through the first attachment moiety; and
(c) a sequencing primer wherein the sequencing primer is an oligonucleotide selected from among oligonucleotides complementary with at least a portion of the amplification primer and oligonucleotides complementary with a portion of the target nucleic acid polymer which is located between the 3' end of the amplification primer and a region of interest in the target nucleic acid polymer.

17. A kit according to claim 16, wherein the attachment moiety and the attachment site are components of an affinity pair.

18. A kit according to claim 17, wherein the affinity pair is selected from the group consisting of biotin/avidin, biotin/streptavidin, poly-dA/poly-dT, poly-dG/poly-dC, hapten/antibody and metal/chelate.

19. A kit according to any one of claims 16 to 18, wherein the attachment moiety does not form a bond directly with the attachment site, further comprising a linking moiety selected so as to form a bond between the attachment moiety and the attachment site.

20. A kit according to any one of claims 16 to 19, wherein the sequencing primer further comprises a detectable label.

21. A kit according to any one of claims 16 to 20, further comprising a reference means for comparison of the determined sequence of the target nucleic acid polymer with the sequence of a standard.

22. A kit according to claim 21, wherein the reference means is a package insert showing the sequencing ladder obtained from sequencing of the standard.

23. A kit according to claim 21, wherein the reference means is a sample of the standard.

24. A kit according to any one of claims 16 to 19 or 21 to 23, wherein the oligonucleotide of the amplification primer is complementary to and hybridizes with a portion of the target nucleic acid polymer which is derived from a target organism.

25. A kit according to any one of claims 16 to 24, further comprising a second amplification primer comprising an oligonucleotide which is complementary to and hybridizes with a portion of the second strand of the target nucleic acid polymer and which is effective as a primer for enzymatic nucleic acid polymerization and a second attachment moiety different from the first attachment moiety; and a plurality of second supports each comprising a solid matrix and at least one attachment site which is capable of immobilizing the oligonucleotide of the amplification probe through the second attachment moiety.

26. A kit according to claim 25, wherein the attachment moiety and the attachment site are components of an affinity pair.

27. A kit according to claim 25 or 26 wherein the affinity pair is selected from the group consisting of biotin/avidin, biotin/streptavidin, poly-dA/poly-dT, poly-dG/poly-dC, hapten/antibody and metal/chelate.

28. A kit according to any one of claims 25 to 27, further comprising a reference means for comparison of the determined sequence of the target nucleic acid polymer with the sequence of a standard.

29. The method according to anyone of claims 1 to 15 for determining in the target nucleic acid polymer at least one potential nucleotide variation.

30. The method according to claim 29 for forensic analysis or for the analysis of hereditary predispositions or diseases.

31. The method according to claim 29 or 30 wherein the nucleotide variation is a somatic mutation.

32. The method according to claim 29 or 30 for the selection of cells and strains or species for plant and animal breeding or for the selection of microorganisms displaying a desired genotype.

33. The kit according to anyone of claims 16 to 28 for use in a method according to anyone of claims 29 to 32.

## Patentansprüche

1. Verfahren zur Bestimmung der Sequenz eines Ziel-Nucleinsäurepolymers, umfassend:
(a) Kombinieren einer das Ziel-Nucleinsäurepolymer enthaltenden Probe mit einem Gemisch aus Nucleosidtriphosphaten, einer Nucleinsäurepolymerase und ersten und zweiten Amplifikationsprimern, wobei jeder Amplifikationsprimer ein Oligonucleotid mit einem Bereich umfaßt, der zu einem unterschiedlichen Strang des Ziel-Nucleinsäurepolymers komplementär ist und damit hybridisieren kann und als ein Primer zur Nucleinsäurepolymerisation durch die Nucleinsäurepolymerase wirksam ist, zur Bildung eines Amplifikationsgemisches, wobei der erste Amplifikationsprimer außerdem eine erste, an das Oligonucleotid gebundene Verknüpfungseinheit umfaßt;
(b) wiederholte Durchführung von Zyklen mit dem Amplifikationsgemisch zwischen einem ersten Zustand, unter dem DNA-Polymerisation eintritt, und einem zweiten Zustand, unter dem eine Denaturierung der doppelsträngigen DNA zu einzelsträngiger DNA eintritt, wobei die Anzahl von Zyklen zur Herstellung von Kopien des Ziel-Nucleinsäurepolymers in einer zur Verwendung in einem DNA-Sequenzierungsverfahren geeigneten Menge ausreichend ist, zur Bildung einer amplifizierten Probe, die Kopien der an die Verknüpfungseinheit gebundenen Ziel-Nucleinsäuresequenz umfaßt;
(c) Zugabe mehrerer erster Träger zu der amplifizierten Probe, wobei jeder eine feste Matrix und eine erste Verknüpfungsstelle umfaßt, die über die erste Verknüpfungseinheit einen Strang des Ziel-Nucleinsäurepolymers immobilisieren kann;
(d) Aufeinanderwirkenlassen der ersten Verknüpfungsstellen mit den ersten Verknüpfungseinheiten, so daß die Kopien der Ziel-Nucleinsäure in der amplifizierten Probe auf den ersten Trägern immobilisiert werden;
(e) Abtrennen der immobilisierten Nucleinsäuren von dem Amplifikationsgemisch;
(f) Denaturieren der amplifizierten Nucleinsäureprobe zur Bildung einzelsträngiger Nucleinsäuren; und
(g) Bestimmung der Sequenz der immobilisierten einzelsträngigen Nucleinsäuren.

2. Verfahren nach Anspruch 1, wobei der Schritt der Denaturierung der amplifizierten Probe vor der Immobilisierung der Kopien der Ziel-Nucleinsäure auf dem Träger durchgeführt wird.

3. Verfahren nach Anspruch 1 oder 2, wobei die Sequenz der einzelsträngigen immobilisierten Nucleinsäuren unter Verwendung eines Kettenabbruchverfahrens bestimmt wird.

4. Verfahren nach einem der Ansprüche 1 bis 3, wobei die erste Verknüpfungseinheit und die erste Verknüpfungsstelle Bestandteile eines Affinitiätspaars sind.

5. Verfahren nach Anspruch 4, wobei das Affinitätspaar ausgewählt ist aus Biotin/Avidin, Biotin/Streptavidin, poly-dA/poly-dT, poly-dG/poly-dC, Hapten/Antikörper oder Metall/Chelat.

6. Verfahren nach einem der Ansprüche 1 bis 5, wobei der zweite Amplifikationsprimer eine zweite Verknüpfungseinheit umfaßt, die sich von der ersten Verknüpfungseinheit unterscheidet.

7. Verfahren nach Anspruch 6, außerdem umfassend den Schritt der Zugabe mehrerer zweiter Träger zu der amplifizierten Probe, die eine Trägermatrix und eine zweite Verknüpfungsstelle umfassen, die einen Strang des Ziel-Nucleinsäurepolymers über die zweite Verknüpfungseinheit immobilisieren kann.

8. Verfahren nach Anspruch 6 oder 7, wobei die zweite Verknüpfungseinheit und die zweite Verknüpfungsstelle Bestandteile eines Affinitätspaars sind.

9. Verfahren nach Anspruch 8, wobei das Affinitätspaar ausgewählt ist aus Biotin/Avidin, Biotin/Streptavidin, poly-dA/poly-dT, poly-dG/poly-dC, Hapten/Antikörper oder Metall/Chelat.

10. Verfahren nach einem der Ansprüche 6 bis 9, wobei die ersten und zweiten Träger gleichzeitig in der amplifizierten Probe vorhanden sind und so ausgewählt sind, daß sie physisch voneinander getrennt werden können.

11. Verfahren nach Anspruch 10, wobei einer der ersten und zweiten Träger magnetisch ist.

12. Verfahren nach Anspruch 10, wobei einer oder beide ersten und zweiten Träger Eintauchstäbe sind.

13. Verfahren nach einem der Ansprüche 1 bis 12, wobei das Verfahren der Sequenzbestimmung folgende Schritte umfaßt:
Kombinieren der einzelsträngigen immobilisierten Nucleinsäuren mit einem Sequenzierungsprimer und einem Squenzierungsgemisch, das Nucleosidtriphosphate umfaßt, ein Kettenabbruchnucleotid, ein Polynucleotid synthetisierendes Enzym und eine Markierungseinheit, die während der Nucleinsäuresynthese eingebaut wird;
Ablaufenlassen der Polymerisation über einen Zeitraum, der ausreicht, um genügend Kettenabbruchereignisse geschehen zu lassen, damit die Sequenz bestimmt werden kann, zur Bildung einer teilweise polymerisierten Probe;
Zugabe eines Reagenzes zur Vervollständigung der Reaktion, das ein Gemisch aus Nucleosidtriphosphaten umfaßt, zu der teilweise polymerisierten Probe;
Ablaufenlassen der Polymerisation in dem Reagens zur Vervollständigung der Reaktion über eine Zeitdauer, die ausreichend ist, um die DNA-Synthese im wesentlichen abzuschließen;
Abtrennen der vollständig polymerisierten Probe von dem Reagens zur Vervollständigung der Reaktion;
Freisetzen der polymerisierten Nucleinsäuren von dem Träger;
Denaturieren der polymerisierten Nucleinsäuren, um diese einzelsträngig zu machen;
Analysieren der Größe der einzelsträngigen Nucleinsäurepolymere zur Bestimmung der Sequenz des Ziel-Nucleinsäurepolymers.

14. Verfahren nach Anspruch 13, wobei der Sequenzierungsprimer ein Oligonucleotid ist, das ausgewählt ist aus Oligonucleotiden, die zu mindestens einem Bereich des Amplifikationsprimers komplementär sind, und Oligonucleotiden, die zu einem Bereich des Ziel-Nucleinsäurepolymers komplementär sind, der zwischen dem 3'-Ende des Amplifikationsprimers und einem gewünschten Bereich in dem Ziel-Nucleinsäurepolymer liegt.

15. Verfahren nach einem der Ansprüche 1 bis 14, außerdem umfassend die Schritte der Inkubation des Amplifikationsgemisches bei einer Temperatur, die so ausgewählt ist, daß sie das Aneinanderlagern der Nucleinsäuren vor jedem Polymerisationsschritt fördert.

16. Kit zur Verwendung bei der Bestimmung der Sequenz eines Ziel-Nucleinsäurepolymers, umfassend eine verpackte Kombination aus
(a) einem ersten Amplifikationsprimer, der ein Oligonucleotid umfaßt, das zu einem Bereich des Ziel-Nucleinsäurepolymers komplementär ist und damit hybridisieren kann, und der als ein Primer für eine enzymatische Nucleinsäurepolymerisation wirksam ist, und einer ersten Verknüpfungseinheit;
(b) mehreren ersten Trägern, die jeweils eine feste Matrix umfassen und mindestens eine Verknüpfungsstelle, die die Amplifikationssonde über die erste Verknüpfungseinheit immobilisieren kann; und
(c) einem Sequenzierungsprimer, wobei der Sequenzierungsprimer ein Oligonucleotid ist ausgewählt aus Oligonucleotiden, die zu mindestens einem Bereich des Amplifikationsprimers komplementär sind, und Oligonucleotiden, die zu einem Bereich des Ziel-Nucleinsäurepolymers komplementär sind, der zwischen dem 3'-Ende des Amplifikationsprimers liegt und einem gewünschten Bereich des Ziel-Nucleinsäurepolymers.

17. Kit nach Anspruch 16, wobei die Verknüpfungseinheit und die Verknüpfungsstelle Bestandteile eines Affinitätspaars sind.

18. Kit nach Anspruch 17, wobei das Affinitätspaar ausgewählt ist aus Biotin/Avidin, Biotin/Streptavidin, poly-dA/poly-dT, poly-dG/poly-dC, Hapten/Antikörper oder Metall/Chelat.

19. Kit nach einem der Ansprüche 16 bis 18, wobei die Verknüpfungseinheit keine direkte Bindung mit der Verknüpfungsstelle bildet, außerdem eine Bindungseinheit umfassend, die so ausgewählt ist, daß sie eine Bindung zwischen der Verknüpfungseinheit und der Verknüpfungsstelle bilden kann.

20. Kit nach einem der Ansprüche 16 bis 19, wobei der Sequenzierungsprimer außerdem eine nachweisbare Markierung umfaßt.

21. Kit nach einem der Ansprüche 16 bis 20, außerdem umfassend ein Referenzmittel zum Vergleich der bestimmten Sequenz des Ziel-Nucleinsäurepolymers mit der Sequenz eines Standards.

22. Kit nach Anspruch 21, wobei das Referenzmittel eine Packungsbeilage ist, die die durch die Sequenzierung des Standards erhaltene Sequenzierungsleiter zeigt.

23. Kit nach Anspruch 21, wobei das Referenzmittel eine Standardprobe des Standards ist.

24. Kit nach einem der Ansprüche 16 bis 19 oder 21 bis 23, wobei das Oligonucleotid des Amplifikationsprimers zu einem Bereich des Ziel-Nucleinsäurepolymers komplementär ist, der von einem Zielorganismus stammt, und damit hybridisiert.

25. Kit nach einem der Ansprüche 16 bis 24, außerdem umfassend einen zweiten Amplifikationsprimer, der ein Oligonucleotid umfaßt, das zu einem Bereich des zweiten Strangs des Ziel-Nucleinsäurepolymers komplementär ist und damit hybridisiert und als ein Primer zur enzymatischen Nucleinsäurepolymerisation wirksam ist, und eine sich von der ersten Verknüpfungseinheit unterscheidende zweite Verknüpfungseinheit; und mehrere zweite Träger, jeweils eine festere Matrix umfassend und mindestens eine Verknüpfungsstelle, die das Oligonucleotid der Amplifikationssonde über die zweite Verknüpfungseinheit immobilisieren kann.

26. Kit nach Anspruch 25, wobei die Verknüpfungseinheit und die Verknüpfungsstelle Bestandteile eines Affinitätspaars sind.

27. Kit nach Anspruch 25 oder 26, wobei das Affinitätspaar ausgewählt ist aus Biotin/Avidin, Biotin/Streptavidin, poly-dA/poly-dT, poly-dG/poly-dC, Hapten/Antikörper oder Metall/Chelat.

28. Kit nach einem der Ansprüche 25 bis 27, außerdem umfassend ein Referenzmittel zum Vergleich der bestimmten Sequenz des Ziel-Nucleinsäurepolymers mit der Sequenz eines Standards.

29. Verfahren nach einem der Ansprüche 1 bis 15 zur Bestimmung mindestens einer potentiellen Nucleinsäurevariation in dem Ziel-Nucleinsäurepolymer.

30. Verfahren nach Anspruch 29 für gerichtsmedizinische Analysen oder für die Analyse von erblichen Prädispositionen oder Krankheiten.

31. Verfahren nach Anspruch 29 oder 30, wobei die Nucleotidvariation eine somatische Mutation ist.

32. Verfahren nach Anspruch 29 oder 30 zur Auswahl von Zellen und Stämmen oder Arten zur Züchtung von Pflanzen und Tieren oder zur Auswahl von Mikroorganismen, die einen gewünschten Genotyp zeigen.

33. Kit nach einem der Ansprüche 16 bis 28 zur Verwendung in einem Verfahren nach einem der Ansprüche 29 bis 32.

## Revendications

1. Procédé de détermination de la séquence d'un polymère d'acide nucléique cible, comprenant :
(a) la combinaison d'un échantillon contenant le polymère d'acide nucléique cible avec un mélange de nucléosidetriphosphates, un acide nucléique polymérase et des première et deuxième amorces d'amplification, chaque amorce d'amplification comprenant un oligonucléotide ayant une région qui est complémentaire et qui s'hybride à un brin différent du polymère d'acide nucléique cible et étant efficace comme amorce pour la polymérisation en acide nucléique par l'acide nucléique polymérase afin de former un mélange d'amplification, dans lequel la première amorce d'amplification contient une première fraction d'assemblage fixée à l'oligonucléotide;
(b) l'introduction, de manière répétée, du mélange d'amplification dans un cycle entre un premier état dans lequel la polymérisation de l'ADN se produit et un deuxième état dans lequel la dénaturation de l'ADN double brin en ADN simple brin se produit, sur un nombre de cycles suffisant pour produire des copies du polymère d'acide nucléique cible en une quantité appropriée pour pouvoir être utilisée dans un processus de séquençage d'ADN, afin de préparer un échantillon amplifié comprenant des copies de la séquence d'acide nucléique cible fixées à la fraction d'assemblage;
(c) l'addition à l'échantillon amplifié de plusieurs premiers supports, chacun composé d'une matrice solide et d'un premier site d'assemblage qui est capable d'immobiliser un brin du polymère d'acide nucléique cible par la première fraction d'assemblage;
(d) l'interaction des premiers sites d'assemblage avec les premières fractions d'assemblage, de sorte que les copies de l'acide nucléique cible dans l'échantillon amplifié soient immobilisées sur les premiers supports;
(e) la séparation des acides nucléiques immobilisés du mélange d'amplification;
(f) la dénaturation de l'échantillon d'acide nucléique amplifié pour former des acides nucléiques simple brin, et
(g) la détermination de la séquence des acides nucléiques simple brin immobilisés.

2. Procédé suivant la revendication 1, dans lequel l'étape de dénaturation de l'échantillon amplifié est réalisée avant l'immobilisation des copies de l'acide nucléique cible sur le support.

3. Procédé suivant la revendication 1 ou 2, dans lequel la séquence des acides nucléiques simple brin immobilisés est déterminée au moyen d'un procédé par terminaison de chaîne.

4. Procédé suivant l'une quelconque des revendications 1 à 3, dans lequel la première fraction d'assemblage et le premier site d'assemblage sont les composants d'une paire d'affinité.

5. Procédé suivant la revendication 4, dans lequel la paire d'affinité est choisie dans le groupe consistant en biotine/avidine, biotine/streptavidine, poly-dA/poly-dT, poly-dG/poly-dC, haptène/anticorps et métal/ chélate.

6. Procédé suivant l'une quelconque des revendications 1 à 5, dans lequel la deuxième amorce d'amplification comprend une deuxième fraction d'assemblage, différente de la première fraction d'assemblage.

7. Procédé suivant la revendication 6, comprenant, en outre, l'étape d'addition à l'échantillon amplifié de plusieurs deuxièmes supports constitués d'une matrice support et d'un deuxième site d'assemblage capable d'immobiliser l'un des brins du polymère d'acide nucléique cible au moyen de la deuxième fraction d'assemblage.

8. Procédé suivant la revendication 6 ou 7, dans lequel la deuxième fraction d'assemblage et le deuxième site d'assemblage sont les composants d'une paire d'affinité.

9. Procédé suivant la revendication 8, dans lequel la paire d'affinité est choisie dans le groupe consistant en biotine/avidine, biotine/streptavidine, poly-dA/poly-dT, poly-dG/poly-dC, haptène/anticorps et métal/ chélate.

10. Procédé suivant l'une quelconque des revendications 6 à 9, dans lequel les premier et deuxième supports sont présents simultanément dans l'échantillon amplifié et sont choisis de manière à être physiquement séparables l'un de l'autre.

11. Procédé suivant la revendication 10, dans lequel l'un des premier et deuxième supports est magnétique.

12. Procédé suivant la revendication 10, dans lequel l'un ou les deux des premier et deuxième supports sont une bâtonnet d'immersion.

13. Procédé suivant l'une quelconque des revendications 1 à 12, dans lequel le procédé de détermination de séquence comprend les étapes de :
combinaison des acides nucléiques simple brin immobilisés avec une amorce de séquençage et un mélange de séquençage constitué de nucléosidetriphosphates, d'un nucléotide de terminaison de chaîne, d'une enzyme synthétisant un polynucléotide et d'une fraction de marquage qui est incorporée pendant la synthèse d'acide nucléique;
l'exécution de la polymérisation pendant une période de temps suffisante pour que suffisamment de phénomènes de terminaison de chaîne se produisent afin de permettre la détermination de la séquence par formation d'un échantillon partiellement polymérisé;
l'addition d'un réactif d'achèvement comprenant un mélange de nucléosidetriphosphates à l'échantillon partiellement polymérisé;
l'exécution de la polymérisation dans le réactif d'achèvement pendant une période de temps suffisante pour sensiblement achever la synthèse de l'ADN;
la séparation de l'échantillon complètement polymérisé du réactif d'achèvement;
la libération des acides nucléiques polymérisés depuis le support;
la dénaturation des acides nucléiques polymérisés pour les rendre simple brin;
l'analyse de la taille des polymères d'acide nucléique simple brin pour déterminer la séquence du polymère d'acide nucléique cible.

14. Procédé suivant la revendication 13, dans lequel l'amorce de séquençage est un oligonucléotide choisi parmi les oligonucléotides complémentaires d'au moins une partie de l'amorce d'amplification et les oligonucléotides complémentaires d'une partie du polymère d'acide nucléique cible, qui est placée entre l'extrémité 3' de l'amorce d'amplification et un région intéressante du polymère d'acide nucléique cible.

15. Procédé suivant l'une quelconque des revendications 1 à 14, comprenant en outre, l'étape d'incubation du mélange d'amplification à une température choisie pour favoriser l'annelage des acides nucléiques avant chaque étape de polymérisation.

16. Trousse à utiliser dans la détermination de la séquence d'un polymère d'acide nucléique cible, comprenant sous forme emballée :
(a) une première amorce d'amplification comportant un oligonucléotide qui est complémentaire et qui s'hybride à une partie du polymère d'acide nucléique cible et qui est efficace comme amorce pour la polymérisation enzymatique de l'acide nucléique et une première fraction d'assemblage;
(b) plusieurs premiers supports, chacun comprenant une matrice solide et au moins un site d'assemblage qui est capable d'immobiliser l'oligonucléotide de l'échantillon d'amplification par la première fraction d'assemblage, et
(c) une amorce de séquençage où l'amorce de séquençage est un oligonucléotide choisi parmi les oligonucléotides complémentaires d'au moins une partie de l'amorce d'amplification et les oligonucléotides complémentaires d'une partie du polymère d'acide nucléique cible, qui est placée entre l'extrémité 3' de l'amorce d'amplification et la région intéressante du polymère d'acide nucléique cible.

17. Trousse suivant la revendication 16, dans lequel la fraction d'assemblage et le site d'assemblage sont les composants d'une paire d'affinité.

18. Trousse suivant la revendication 17, dans lequel la paire d'affinité est choisie dans le groupe consistant en biotine/avidine, biotine/streptavidine, poly-dA/poly-dT, poly-dG/poly-dC, haptène/anticorps et métal/chélate.

19. Trousse suivant l'une quelconque des revendications 16 à 18, dans lequel la fraction d'assemblage ne forme pas de lien direct avec le site d'assemblage, comprenant en outre, une fraction de liaison choisie de manière à former un lien entre la fraction d'assemblage et le site d'assemblage.

20. Trousse suivant l'une quelconque des revendications 16 à 19, dans lequel l'amorce de séquençage comprend en outre, un marqueur détectable.

21. Trousse suivant l'une quelconque des revendications 16 à 20, comprenant en outre, un dispositif de référence pour comparer la séquence déterminée du polymère d'acide nucléique cible avec la séquence d'un témoin.

22. Trousse suivant la revendication 21, dans lequel le dispositif de référence est un article emballé présentant l'échelle de séquençage obtenue par le séquençage du témoin.

23. Trousse suivant la revendication 21, dans lequel le dispositif de référence est un échantillon du témoin.

24. Trousse suivant l'une quelconque des revendications 16 à 19 ou 21 à 23, dans lequel l'oligonucléotide de l'amorce d'amplification est complémentaire et s'hybride à une partie du polymère d'acide nucléique cible qui provient d'un organisme cible.

25. Trousse suivant l'une quelconque des revendications 16 à 24, comprenant en outre, une deuxième amorce d'amplification comportant un oligonucléotide qui est complémentaire et qui s'hybride à une partie du deuxième brin du polymère d'acide nucléique cible et qui est efficace comme amorce pour la polymérisation enzymatique de l'acide nucléique et une deuxième fraction d'assemblage différente de la première fraction d'assemblage et plusieurs deuxièmes supports, chacun comportant une matrice solide et au moins un site d'assemblage qui est capable d'immobiliser l'oligonucléotide de l'échantillon d'amplification par la deuxième fraction d'assemblage.

26. Trousse suivant la revendication 25, dans lequel la fraction d'assemblage et le site d'assemblage sont les composants d'une paire d'affinité.

27. Trousse suivant la revendication 25 ou 26, dans lequel la paire d'affinité est choisie dans le groupe consistant en biotine/avidine, biotine/streptavidine, poly-dA/poly-dT, poly-dG/poly-dC, haptène/ anticorps et métal/chélate.

28. Trousse suivant l'une quelconque des revendications 25 à 27, comprenant en outre, un dispositif de référence pour comparer la séquence déterminée du polymère d'acide nucléique cible avec la séquence d'un témoin.

29. Procédé suivant l'une quelconque des revendications 1 à 15, pour déterminer au moins une modification potentielle de nucléotide dans le polymère d'acide nucléique cible.

30. Procédé suivant la revendication 29, pour l'analyse légale ou pour l'analyse de prédispositions ou maladies héréditaires.

31. Procédé suivant la revendication 29 ou 30, dans lequel la modification nucléotidique est une mutation somatique.

32. Procédé suivant la revendication 29 ou 30, pour sélectionner des cellules ou souches ou espèces de plantes et d'animaux d'élevage ou pour sélectionner des micro-organismes présentant un génotype souhaité.

33. Trousse suivant l'une quelconque des revendications 16 à 28, à utiliser dans un procédé suivant l'une quelconque des revendications 29 à 32.
